# EUROPÄISCHE PATENTSCHRIFT

(11) **EP 1 185 256 B1**
(45) Veröffentlichungstag und Bekanntmachung des Hinweises auf die Patenterteilung: **06.10.2004**
(21) Anmeldenummer: 00922666.3
(22) Anmeldetag: 27.04.2000
(51) Int. Cl.: A61K 31/025, A61P 27/02

(54) **VERWENDUNG EINES HOCHFLUORIERTEN OLIGOMEREN ALKANS IN DER OPHTHALMOLOGIE**
UTILIZATION OF A HIGHLY FLUORINATED OLIGOMERIC ALKAN IN OPHTHALMOLOGY
UTILISATION D'UN ALCANE OLIGOMERE A HAUTE TENEUR EN FLUOR EN OPHTALMOLOGIE

(30) Priorität: 12.06.1999 DE 19926890
(43) Veröffentlichungstag der Anmeldung: 13.03.2002
(73) Patentinhaber: BAUSCH & LOMB INCORPORATED, Rochester, New York 14604-2701 (US)
(72) Erfinder: GROSS, Udo, D-12683 Berlin (DE); MENZ, Dirk-Henning, D-86420 Diedorf (DE); KEMNITZ, Erhard, D-10405 Berlin (DE); HOERAUF, Hans, D-23730 Sierksdorf (DE); KOBUCH, Karin, D-93080 Pentling (DE)
(74) Vertreter: Maiwald Patentanwalts GmbH
(86) Internationale Anmeldenummer: PCT/EP2000/003817
(87) Internationale Veröffentlichungsnummer: WO 2000/076491

(56) Entgegenhaltungen:
- EP-A- 0 563 446
- EP-A- 0 877 010
- DE-A- 19 536 504
- DE-A- 19 861 012
- US-A- 4 490 351
- KOBUCH, K. A. (1) ET AL: "A new hydrofluorocarbon with increased viscosity and reduced density for long-term tamponade of the lower retina." IOVS, (MARCH 15, 2000) VOL. 41, NO. 4, PP. S662. MEETING INFO.: ANNUAL MEETING OF THE ASSOCIATION IN VISION AND OPTHALMOLOGY. FORT LAUDERLADE, FLORIDA, USA APRIL 30-MAY 05, 2000 ASSOCIATION FOR RESEARCH IN VISION AND OPHTHALMOLOGY., XP000956167
- SANTORO, E. ET AL: "Mass spectra of some partially fluorinated aliphatic compounds" ORG. MASS SPECTROM. (1973), 7(2), 123-31 , 1973, XP002150832
- HOERAUF H ET AL: "[O44--a solvent for silicone oil adhesions on intraocular lenses]. O44--ein Losungsmittel fur Silikonoladhasionen auf Intraokularlinsen." KLINISCHE MONATSBLATTER FUR AUGENHEILKUNDE, (1999 FEB) 214 (2) 71-6. , XP000956155

## Beschreibung

Die Erfindung betrifft die Verwendung eines hochfluorierten oligomeren Alkans zur Herstellung eines Behandlungsmittels für die Anwendung in der Ophtalmologie gemäß Anspruch 1.

Die US-A-4,490,351 beschreibt die Verwendung flüssiger Perfluorcarbone Für die Behandlung und Diagnostik in der Ophthalmologie. Während derartige Substanzen zur temporären Behandlung von Netzhautablösungen und als Glaskörpertamponade eingesetzt werden, ist ihr Langzeiteinsatz, beispielsweise als Glaskörperersatz, mit verschiedenartigen Schädigungen des Auges verbunden. Bekannt sind Nebenwirkungen, beispielsweise von Perfluorpolyethem und Perfluorcarbonen, die auf mechanische Schädigungen an der Netzhaut und am Sehnerv zurückgeführt werden, wobei dafür die große Dichte (ca. 1,9g/cm³) dieser Verbindungen verantwortlich gemacht wird.

In den Druckschriften EP 0 563 446 A1, DE 195 36 504 A1 und DE 197 19 280 A1 werden dagegen teilfluorierte Flüssigkeiten vorgeschlagen, die nur eine Dichte zwischen 1,1 und 1,3g/cm³ aufweisen und die als Hilfsmittel in der Ophthalmologie zur Netzhautentfaltung, zur Glaskörpertamponade und als Glaskörperersatz eingesetzt werden können. Auch diese Substanzen führen trotz ihrer deutlich geringeren Dichten zu histologischen Veränderungen im Bereich der Netzhaut und auch zu ansonsten von perfluorierten Carbonen bekannten Nebenwirkungen.

Schließlich beschreiben die DE 44 05 627 A1 und die EP 05 45 174 A1 Fluorkohlenwasserstoffe enthaltende Oligomere der allgemeinen Formeln (R_{F})ₓ-R_{H} wobei R_{F} einen hochfluorierten Alkylrest bedeutet, der unmittelbar oder über ein Bindeglied an die Gruppe R_{H} gebunden ist, die für einen Alkanrest oder Wasserstoff steht und x eine Zahl von 1 bis 4 bedeutet und aus Verbindungen der allgemeinen Formel Y-(CF₂)a-O_{b}-(CH₂)_{c}-CH=CH₂ hergestellt werden wobei Y für ein Wasserstoff- oder ein Fluoratom steht, a eine Zahl von 2 bis 16 ist und b sowie c - unabhängig voneinander eine Zahl zwischen 0 und 1 sind und d eine Zahl zwischen 0 und 6 bedeutet, mit einem mittleren Oligomerisationsgrad von 2 bis 4. In den genannten Druckschriften wird die Verwendung der beschriebenen Substanzen als Schmier- oder Gleitmittel und als Skibelag sowie als Sauerstoffträger beschrieben.

Es besteht die Aufgabe, ein pharmakologisches Mittel zur Anwendung in der Ophthalmologie bereitzustellen, welches auch bei Anwendung über einen längeren Zeitraum keine oder nur geringfügige Schädigungen der Retina verursacht.

Gelöst wird diese Aufgabe durch die Verwendung einer Substanz gemäß Anspruch 1. Vorteilhafte Verwendungen und Ausgestaltungen sind den Unteransprüchen entnehmbar.

Die der Erfindung zugrundeliegende und einen Teil der Erfindung bildende Erkenntnis besteht darin, daß die schädigende Wirkung von Fluorcarbonen beim Langzeiteinsatz im Auge wesentlich durch ihre Fähigkeit zur Gewebepenetration und durch das Zusammenwirken ihrer geringen Viskositäten und ihrer hohen Dichten hervorgerufen wird. Dies gilt nicht nur bei der Verwendung dieser Stoffe als Glaskörperersatz in der Ophthalmologie, sondern auch bei ihrer Nutzung als flüssige Implantate, als Tränenersatzstoffe und Behandlungsmittel für das Auge unmittelbar oder als Wirkstoffträger.

Diese Erkenntnis führte zur Suche nach Substanzen mit höherer Viskosität bei ausreichend hoher Dichte sowie ausreichender Lösefähigkeit sowie gleichzeitig nur minimaler Gewebepenetration.

Die Gewebepenetrationsfähigkeit läßt sich qualitativ beschreiben durch die kritische Löslichkeitstemperatur in Hexan (Abkürzung: CST). Dieses qualitative Maß für die Lipophilie ist gleichzeitig ein Maß für die Fähigkeit zur Gewebepenetration. Während die niedermolekularen R_{F}-R_{H}-Verbindungen, wie sie aus dem eingangs genannten Stand der Technik auch in der Verwendung für die Ophthalmologie bekannt sind, in der Regel CST-Werte unterhalb von -20° C aufweisen, sind im Gegensatz dazu die hochmolekularen, erfindungsgemäßen Oligomer-Verbindungen erst bei Temperaturen von über +35° C mit Hexan vollständig mischbar und dokumentieren damit ihre Schwer- bzw. Unlöslichkeit in Lipiden, also ihre geringe Gewebepenetrationsfähigkeit. Überraschenderweise besitzen sie trotzdem ein im Vergleich zu ihrem monomeren Analogon erhöhtes Lösevermögen für bestimmte unpolare Substanzen.

Die erfindungsgemäßen Substanzen sind wegen ihrer besonderen Molekülform, trotz sehr ähnlicher chemischer Zusammensetzung mit den bekannten R_{F}-R_{H}-Verbindungen, nicht gewebepenetrierend und daher für den Langzeiteinsatz im Auge geeignet, da erkannt wurde, daß die gewebeschädigenden Eigenschaften der bekannten Substanzen auf deren hohe Penetration zurückzuführen sind. Vorteilhaft für ophthalmologische Anwendungen ist es, daß das erfindungsgemäß verwendete Behandlungsmittel wegen der Kombination von reduzierter Dichte und erhöhter Viskosität nicht mechanisch schädigend im Auge wirkt und deshalb als Langzeitglaskörperersatz verwendet werden kann. Im Vergleich zum derzeit verwendeten Silikonöl hat die erfindungsgemäße Verwendung der Oligomere den Vorteil, daß sie für eine abgestimmte Sauerstofftherapie für die ischämische Retina und eine gleichzeitig vorzügliche Tamponadewirkung durch die besonderen Oberflächeneigenschaften und ihrer im Vergleich zu Silikonöl und Wasser höheren Dichte auch im unteren Augensegment einsetzbar sind.

Aus dem gleichen Grund ermöglicht die erfindungsgemäße Verwendung der beschriebenen Oligomere verschiedene weitere ophthalmologische Anwendungen, die bisher ausschließlich Silikonölen vorbehalten waren. Insbesondere betrifft dies die Verwendung als Volumenersatzstoff, z. B. in Form von Implantaten. Darüberhinaus wurden jedoch auch völlig neue ophthalmologische Anwendungen möglich. Bei der erfindungsgemäßen Verwendung der Oligomere in einer besonders viskosen Ausführung können getrübte, natürliche Linsen ersetzt werden. Dabei erfolgt die Extraktion der natürlichen Linse unter Erhalt des sie umgebenden Kapselsacks, die von einer Injektion des Hochviskosenoligomers in diesen Kapselsack gefolgt wird. Die fluiden Linsenmaterialien ermöglichen den Erhalt der Akkomodation. Vorteilhafterweise können dafür Oligomere mit starkem Verzweigungsgrad verwendet werden, oder es wird die an sich schon hohe Viskosität der Oligomere noch weiter gesteigert, indem durch Zusatz von bestimmten Mischungen von Wasser und oberflächenaktiven Substanzen nach bekannten Verfahren Gele erzeugt werden.

Zusätzlich wird dadurch eine Aufnahme wasserlöslicher Stoffe in die Flüssigimplantate ermöglicht, was deren therapeutische Anwendbarkeit wesentlich erweitert. So sind mit diesen Gelen Anwendungen möglich geworden, bei denen es auf extrem hohe Viskosität (fluide Linsen) und geringstmögliche mechanische Belastung angrenzenden Gewebes bei gleichzeitiger Aufnahmefähigkeit für Ionen (Glaskörperersatz) ankommt. Ein ganz wesentlicher Vorteil im Vergleich zu üblichen Fluorcarbongelen besteht darin, daß selbst bei Zerstörung der Gele die zurückbleibenden Medien die Implantatwirkungen teilweise aufrecht erhalten können, weil sie selbst schon eine ausreichende Viskosität besitzen.

Die Verwendung als Wirkstoffträger basiert auf der Möglichkeit zur Auflösung von pharmakologischen Wirkstoffen in den Oligomeren, wobei besonders die ungewöhnliche Kombination von Lösefähigkeit einerseits und unterdrückter Gewebepenetration andererseits von Bedeutung ist. Deshalb ist die Verwendung als Wirkstoffträger sowohl in Form von Volumenersatzstoffen als auch ohne diese Anwendungsfunktion von besonderem Interesse.

Die vorteilhaften physikalischen Eigenschaften wie Sauerstofflöslichkeit sowie filmbildende, jedoch nicht gewebepenetrierende Wirkung erlauben weitere Anwendungen der hochfluorierten Oligomere, zum Beispiel als Tränenersatzmittel.

Ein wesentlicher Vorteil der beschriebenen Substanzen im Hinblick auf die erfindungsgemäße Verwendung besteht darin, daß diese die bekannten Eigenschaften von Fluorcarbonen und Silikonöl miteinander vereinen, insbesondere die hohe Viskosität von Silikonöl und die hohe Dichte und Sauerstofflöslichkeit von Fluorcarbonen, und zwar ohne die von anderen bekannten Stoffen, beispielsweise Fluorsiloxanen, bekannten Toxizitäten oder die komplizierten Verhältnisse, die bei Mischungen von Silikonöl und teilfluorierten Alkanen vorliegen und ihre Verwendung als Behandlungsmittel in der Ophthalmologie einschränken oder ausschließen.

Zusätzlich bieten die Oligomere bei ihrer ophthalmologischen Nutzung den Vorteil, daß ihre Entfernung im Gegensatz zu den Silikonölen durch problemloses Auswaschen gelingt, da mit den in der ophthalmologischen Praxis für Kurzzeitanwendung bewährten Perfluorcarbonen, zum Beispiel Perfluordecalin oder Perfluoroctan, aber auch mit den teilfluorierten Alkanen wie zum Beispiel C₆F₁₃C₂H₅ oder C₄F₉C₄H₉ vorzügliche bioverträgliche Lösemittel zur Verfügung stehen. Andererseits kann bei Bedarf auch ein Austausch gegen Silikonöl oder umgekehrt erfolgen, ohne daß die Verbindungen sich dabei vermischen.

Die beschriebenen oligomeren Verbindungen haben eine viskose bis hochviskose oder sogar pastöse Konsistenz und eine Dichte zwischen 1,2 und 1,7 g/cm³. Sie sind optisch transparent mit einem Brechungsindex zwischen 1,30 und 1,45. Die Verbindungen sind im wesentlichen unpolar. Jedoch ergibt sich als Besonderheit, verglichen mit den beiden Molekülbestandteilen Alkan und Perfluoralkan, im isolierten Zustand eine schwache charakteristische Molekülpolarität durch die verbindungstypische CF₂-CH₂-Gruppierung. Die stoffbedingten physikalischen Eigenschaften wie Molmasse, Dichte, Brechungsindex, Viskosität, optische Transparenz und das Löslichkeitsverhalten lassen sich, dem Anwendungszweck folgend, variieren. Dies geschieht durch die Wahl der R_{F}- und R_{H}-Gruppierungen, ihres Verzweigungsgrads und des Oligomerisierungsgrads. Die physiko-chemischen Eigenschaften lassen sich aber auch durch Zusatz von perfluorierten oder teilfluorierten Alkannen einstellen. Die hochfluorierten Oligomere sind chemisch und thermisch außergewöhnlich stabil und enzymatisch nicht abbaubar. Sie sind toxikologisch und dermatologisch unbedenklich.

Die Herstellung und Reinigung der beschriebenen Substanzen erfolgt in an sich bekannter Weise. Beispielsweise werden diese wie in der DE 41 39 765 beschrieben hergestellt oder durch Reaktion eines verzweigten, perfluorierten Olefins mit Kalium- oder Cäsiumfluorid in einem aprotischen Lösungsmittel mit einem Alkenylhalogenid und Oligomerisierung mit einem Radikalinitiator in einem unpolaren Lösungsmittel erhalten. Die Reinigung erfolgt beispielsweise durch extraktive und säulenchromatographische Verfahren.

Einige Ausführungsbeispiele für ein hochfluoriertes Oligomer werden im folgenden näher beschrieben.

### Beispiel 1 (nicht erfindungsgemäß)

Das hochfluorierte Oligomer (C₆F₁₃CHCH₂)₃ wird, in Fluorcarbonen gelöst, über eine Al₂O₃-Chromatographiesäule gereinigt. Nach Entfernen des Lösungsmittels werden im Vakuum letzte Spuren flüchtiger Verunreinigungen entfernt. Das hochfluorierte Trimer ist nach Untersuchungen durch Kernspinresonanz, Infrarot- und Massenspektroskopie eine einheitliche und hochreine Substanz mit einem Molekulargewicht von 1055. Die Beladung mit einem Wirkstoff erfolgt schonend über ein nicht mit dem Trimer mischbares Lösungsmittel nach Verteilung bis zur Sättigungskonzentration. β-Carotin als Wirkstoff färbt dabei das Oligomer schwach an. Bei 121° C wird im Autoklaven sterilisiert, ohne daß die physikalischen oder chemischen Eigenschaften der Substanz sich ändern. Die kernmagnetischen Daten dieser Substanz lauten wie folgt:
¹H-NMR:CH₃:0,95ppm;-CH-CF₂-:2,02ppm;CH₂-:1,64ppm
¹⁹F-NMR: CF₃-:82,8ppm;-CH-CF₂:-116,2ppm;4CF₂:-123 ,2ppm,-124,3ppm,-124, 8ppm,-127, 7ppm
¹³C-NMR:CH₃-/CH₂-:25-35ppm
   CF₃-/CF₂-:105-120ppm.

Die Reinheit der Substanz ist wesentlich durch die nachfolgenden physikalischen Eigenschaften charakterisiert:
Dichte: 1,661 g/cm³;
Brechungsindex n^{D} ₂₀: 1,3351;
Oberflächenspannung 19,5 mN/m;
kritische Löslichkeitstemperatur in n-Hexan CST: 35°C;
Viskosität: 220 mPas.

Dieses Oligomer eignet sich für sämtliche oben und in den Ansprüchen beschriebene Verwendungen und weist die oben genannten Vorteile, insbesondere im Langzeitbereich auf.

### Beispiel 2

72 g Tridekafluoro-4,4-dimethylhepten-1 wird gelöst in 100 ml n-Hexan und mit 1,7g Di-tert butylperoxid bei 190°C im Autoklaven oligomerisiert, wobei ein Reaktionsgemisch aus einer dimeren und trimeren Verbindung resultiert. Das Gemisch wird destillativ getrennt, wobei das Trimere als viskoses farbloses Öl der Zusammensetzung [CF₃ CF₂ CF₂ C(CF₃)₂ CH₂ CH CH₂]₃ mit einer durchschnittlichen Molmasse von 1085 Dalton erhalten wird. Gemäß ¹⁹F- und ¹H-NMR sowie infrarotdektroskopischen Untersuchungen entspricht die Verbindung der allgemeinen Formel der Erfindung und bestätigt zudem einen hohen Reinheitsgrad. Entsprechend den relevanten in vitro-Tests ist das hochfluorierte Öl für die beanspruchten Verwendungen geeignet.

## Patentansprüche

1. Verwendung eines hochfluorierten oligomeren Alkans zur Herstellung eines Behandlungsmittels in der Ophthalmologie oder in pharmakologischen Zubereitungen für die Ophthalmologie, wobei das hochfluorierte oligomere Alkan hergestellt wird durch Lösen von Tridekafluoro-4,4-dimethylhepten-1 in n-Hexan und Umsetzen mit Di-*tert*-butylperoxid bei 190°C im Autoklaven, sowie nachfolgende destillative Trennung des Reaktionsgemisches zur Gewinnung des Trimeren des hochfluorierten oligomeren Alkans.

2. Verwendung nach Anspruch 1 als Glaskörperersatz und/oder zur Netzhauttamponade.

3. Verwendung nach Anspruch 1 als flüssiges Implantat.

4. Verwendung nach Anspruch 1 als Tränenersatzstoff.

5. Verwendung nach Anspruch 1 als Trägersubstanz für einzubringende pharmakologische Wirkstoffe.

6. Verwendung nach einem der voranstehenden Ansprüche, **dadurch gekennzeichnet, dass** das oligomere Alkan als ein Bestandteil pharmakologischer Zubereitungen für die Ophthalmologie und damit in Mischungen angewendet wird.

## Claims

1. The use of a highly fluorinated oligomeric alkane for the manufacture of a treatment agent for ophthalmology or in pharmacological preparations for ophthalmology, wherein the highly fluorinated oligomeric alkane is prepared by dissolving tridekafluoro-4,4-dimethylheptene-1 in n-hexane and reacting with di-tert-butylperoxide at 190°C in an autoclave, and subsequent separation of the reaction mixture by destination in order to obtain the trimer of the highly fluorinated oligomeric alkane.

2. The use according to claim 1, as a vitreous body substitute and/or for retina tamponade.

3. The use according to claim 1, as a liquid implant.

4. The use according to claim 1, as a tear replacement.

5. The use according to claim 1, as a carrier substance for pharmacological agents to be administered.

6. The use according to one of the previous claims,
**characterized in that** the oligomeric alkane is used as a component of pharmacological preparations for ophthalmology, and is therefore used in mixtures.

## Revendications

1. Utilisation d'un alcane oligomère à haute teneur en fluor pour préparer un produit de traitement en ophtalmologie ou dans des préparations pharmacologiques en ophtalmologie, dans laquelle l'alcane oligomère à haute teneur en fluor est préparé en dissolvant du tridécafluoro-4,4-diméthylhept-1-ène dans du n-hexane et en le faisant réagir avec du peroxyde de di-*tert*-butyle à 190°C dans un autoclave et ensuite en séparant le mélange réactionnel par distillation afin d'obtenir le trimère de l'alcane oligomère à haute teneur en fluor.

2. Utilisation selon la revendication 1 en tant que remplacement du corps vitré et/ou pour le tamponnement de la rétine.

3. Utilisation selon la revendication 1 en tant qu'implant liquide.

4. Utilisation selon la revendication 1 en tant que substitut des larmes.

5. Utilisation selon la revendication 1 en tant que substance support pour des principes actifs pharmacologiques à introduire.

6. Utilisation selon l'une des revendications précédentes, **caractérisée en ce que** l'alcane oligomère est utilisé comme constituant de préparations pharmacologiques pour l'ophtalmologie et donc dans des mélanges.
